Europäisches Patentamt

European Patent Office

Office européen des brevets

(11) Publication number: **0 225 639 B1**

(12) **EUROPEAN PATENT SPECIFICATION**

(45) Date of publication of patent specification: 06.03.91      (51) Int. Cl.⁵: **A61K 7/06**

(21) Application number: 86117190.8

(22) Date of filing: 10.12.86

(54) Hair grower composition containing vasoactive intestinal polypeptide as active ingredient and method of promoting hair growth employing same.

(30) Priority: 10.12.85 JP 276099/85

(43) Date of publication of application:
16.06.87 Bulletin 87/25

(45) Publication of the grant of the patent:
06.03.91 Bulletin 91/10

(84) Designated Contracting States:
DE FR GB IT

(56) References cited:
US-A- 3 898 329

(73) Proprietor: MEIJI SEIKA KAISHA LTD.
4-16 Kyobashi 2-chome
Chuo-ku Tokyo 104(JP)

(72) Inventor: Yanaihara, Noboru
403-22, Kita
Shizuoka-shi Shizuoka(JP)
Inventor: Watanabe, Suzuo
2-15-4, Iwato
Yokosuka-shi Kanagawa(JP)
Inventor: Kasai, Michiyuki
Nagao Jutaku 2-406 2-7, Kamikihon-cho
Miyamae-ku Kawasaki-shi Kanagawa(JP)
Inventor: Sato, Toyomi
4-22-10, Higashi Nippori
Arakawa-ku Tokyo(JP)
Inventor: Kikkoji, Toshihiro
1-11-12, Sanno
Ohta-ku Tokyo(JP)

(74) Representative: WILHELMS, KILIAN & PART-
NER Patentanwälte
Eduard-Schmid-Strasse 2
W-8000 München 90(DE)

**Description**

FIELD OF THE INVENTION

This invention relates to a new use for VIP vasoactive intestinal polypeptide (hereinafter "VIP"), a known digestive tract hormone as an active ingredient in a hair grower to promote hair growth.

BACKGROUND OF THE INVENTION

In 1970, VIP was extracted from the intestinal tract and found to cause an increase in blood flow. It was thereafter found that this hormone is a peptide present in both the intestinal tract and the cerebral nervous system. VIP is thought to be a possible adrenergic neurotransmitter (see Shokakan Horumon Kenkyukai (ed.), Shokakan Horumon-no Shinpo 4, Chugai Igakusha (1981)).

VIP is a basic peptide having a molecular weight of 3326 comprising 28 amino acids arranged in the amino acid sequence represented by formula (I) below:

$$His-Ser-Asp-Ala-Val-Phe-Thr-$$

$$Asp-Asn-Tyr-Thr-Arg-Leu-arg- \qquad (I)$$

$$Lys-Gln-Met-Ala-Val-Lys-Lys-$$

$$Tyr-Leu-Asn-Ser-Ile-Leu-Asn-NH_2$$

wherein His, Ser, Asp, Ala, Val, Phe, Thr, Asn, Tyr, Arg, Leu, Lys, Gln, Met, and Ile represent residual groups of histidine, serine, aspartic acid, alanine, valine, phenylalanine, threonine, asparagine, tyrosine, arginine, leucine, lysine, glutamine, methionine, and isoleucine, respectively.

SUMMARY OF THE INVENTION

It has been found in the present invention that VIP, when applied to the skin, causes a local increase in blood flow and promotes hair growth.

DETAILED DESCRIPTION OF THE INVENTION

VIP which can be used in the present invention is commercially available. The VIP may be either a synthetic VIP as described, e.g., in U.S. Patent 3,862,927 or a naturally-occurring VIP as described, e.g., in U.S. Patent 4,119,118.

For topical application of VIP to the skin, various known methods for percutaneous administration, such as a combined use with a cutaneous penetration intensifier, are effectively empolyed. Various dosage forms and methods of formulation suited for topical application of the active ingredient are known (cf. U.S. Patent 4,139,619), and any of them may be used in this invention.

The hair grower compositions formulated by the present invention include fluids and semisolids. The fluids include topically applicable lotions and sprays. As is known in the art, such fluids generally comprise a pharmaceutically acceptable diluent or carrier which can rapidly vaporize while leaving the whole or substantially the whole of the active ingredient at the applied site.

The preferred hair grower compositions according to the present invention are semisolids having a relatively high viscosity which can easily be spread over the entire surface of the skin to be treated without extending themselves beyond the desired region. Examples of such semisolid include ointments, gels, pastes and creams. It is preferable that the semisolid compositions contain a diluent or carrier medium that would rapidly vaporize when applied to thereby help the active ingredient to remain on the skin.

Examples of the volatile diluent or carrier medium to be used in the fluid compositions and semisolid compositions include topically applicable alcohols capable of vaporizing at room temperature, such as alkanols having from 1 to 4 carbon atoms, e.g., ethanol, or isopropanol.

The semisolids that are the preferred dosage forms according to the invention suitably comprise an appropriate base. The bases to be used for semisolids include hydrocarbon oils (e.g., liquid paraffin), absorbent bases (anhydrous) (e.g., hydrophilic petrolatum), W/O emulsion type bases (e.g., lanolin, cold creams), water-soluble bases (e.g., polyethylene glycol), and the like. The amount of the base is preferably 90% by weight or more based on the hair grower composition.

The liquid compositions of the present invention may further contain various vitamins in consideration for nutrition; adjuvants, such as peripheral vasodilators (e.g., acetylcholine derivatives: usually from 1 to 2% by weight based on the composition), keratolytic agents (e.g., salicylic acid: usually from 0.1 to 0.4% by weight based on the composition), agents for skin hyperfunction (e.g., red pepper: usually from 0.1 to 0.5% by weight based on the composition); and other additives, such as moisture-retaining agents (e.g., glycerol, propylene glycol: usually from 1 to 5% by weight based on the composition; hyaluronic acid salt: usually from 0.05 to 1% by weight based on the composition), bacteriocides (e.g., benzalkonium chloride: usually from 0.005 to 0.1% by weight based on the composition; benzethonium chloride: usually from 0.025 to 1% by weight based on the composition), flavoring agents, coloring agents, and the like.

The hair grower composition may usually contain VIP in an amount of from 0.005 to 0.5% by weight based on the composition.

The effective dosage level of VIP for topical application ranges from 1.0 to 0.01 mg/cm²/day, and preferably from 0.5 to 0.05 mg/cm²/day.

The hair grower composition of this invention may be applied preferably 2 to 3 times a day.

This invention will now be illustrated in greater detail with reference to the following non-limiting examples.

## EXAMPLE

A hundred milligrams of VIP (produced by Peptide Kenkyusho K.K.) were added to an emulsion type lotion base consisting of the following components to prepare a 0:1 wt% hair grower lotion.

### Lotion Base Formulation:

| | |
|---|---|
| Bleached bees wax | 0.1 g |
| Cetyl alcohol | 1.5 g |
| Sodium lauryl sulfate | 0.5 g |
| Glycerol | 5.0 g |
| Purified water | 100.0 ml |

## TEST EXAMPLE I

Effect on Skin Temperature Increase in Rabbit (under anesthesia)

The VIP hair grower lotion of Example of a placebo lotion having the same composition as that of Example except for containing no VIP was applied to the back (3 cm x 3 cm) of tied rabbits (Japanese white rabbits; three animals per group). The subcutaneous temperature under the skin to which the lotion was applied was measured with a depth thermometer ("CTM-201" manufactured by Terumo Co.) after one hour from the application. The results obtained are shown in Table I.

## TABLE 2

### Animal Number

| Preparation | 1 | 2 | 3 | Mean | S.E. |
|---|---|---|---|---|---|
| VIP hair grower lotion | 38.8° | 38.5° | 38.2° | 38.5° | ± 0.17 |
| Placebo lotion | 37.5° | 37.0° | 37.3° | 37.3° | ± 015 |

The results of Table I clearly demonstrate that VIP increases body temperature.

TEST EXAMPLE 2

Effect on Hair Growth in Mouse

The hair at the back of 20-week-old $C_3H$ male mice (5 animals per group) was cut off, and 50 $\mu\ell$/dose of the same VIP hair grower lotion or placebo lotion as used in Test Example I was applied thereto 4 times a day for 3 consecutive weeks. On the day following the final application, the mice were anesthetized with ethyl ether. After shaving cream was applied to their backs, the pelage, an area measuring I.5 cm x 2.5 cm, within which the lotion had been applied was shaved off with a safety razor. The collected hair was washed with water in a beaker, filtered, dried at 65°C for 24 hours, allowed to stand in a moist room for 24 hours, and then weighed. The results obtained are shown in Table 2 below.

## TABLE 2

### Amount of Hair (mg)

### Animal Number

| Preparation | 1 | 2 | 3 | 4 | 5 | Mean | S.E. |
|---|---|---|---|---|---|---|---|
| VIP hair brower lotion | 10.09 | 12.55 | 9.71 | 10.58 | 10.93 | 10.77 | ± 0.49 |
| Placebo lotion | 5.31 | 6.26 | 5.32 | 4.48 | 6.01 | 5.48 | ± 0.31 |

From the Student's t test of the data of Table 2, it is clear that the VIP-containing lotion according to the present invention stimulate hair growth with a significant difference of P<0.0I.

## Claims

1. A hair grower composition containing, as an active ingredient, a peptide represented by the formula (I):

His-Ser-Asp-Ala-Val-Phe-Thr-

Asp-Asn-Tyr-Thr-Arg-Leu-arg-                    (I)

Lys-Gln-Met-Ala-Val-Lys-Lys-

Tyr-Leu-Asn-Ser-Ile-Leu-Asn-NH$_2$

4

wherein His, Ser, Asp, Ala, Val, Phe, Thr, Asn, Tyr, Arg, Leu, Lys, Gln, Met, and Ile represent histidine, serine, aspartic acid, alanine, valine, phenylalanine, threonine, asparagine, tyrosine, arginine, leucine, lysine, glutamine, methionine, and isoleucine, respectively, in an amount of from 0,005 to 0,5 % by weight based on the composition and a pharmaceutically acceptable carrier or diluent.

2. The hair grower composition as claimed in Claim I, wherein said carrier is a fluid or semisolid.

3. The hair grower composition as claimed in Claim 2, wherein said carrier is a semisolid.

4. The hair grower composition as claimed in Claim 3, wherein said semisolid is selected from liquid paraffin, hydrophilic petrolatum, lanolin and polyethylene glycol.

5. A method for increasing hair growth comprising topically applying to the skin, a hair grower composition containing a peptide represented by the formula (I):

$$His-Ser-Asp-Ala-Val-Phe-Thr-$$
$$Asp-Asn-Tyr-Thr-Arg-Leu-Arg-\qquad (I)$$
$$Lys-Gln-Met-Ala-Val-Lys-Lys-$$
$$Tyr-Leu-Asn-Ser-Ile-Leu-Asn-NH_2$$

wherein His, Ser, Asp, Ala, Val, Phe, Thr, Asn, Tyr, Arg, Leu, Lys, Gln, Met, and Ile represent histidine, serine, aspartic acid, alanine, valine, phenylalanine, threonine, asparagine, tyrosine, arginine, leucine, lysine, glutamine, methionine, and isoleucine, respectively in an amount of from 0,005 to 0,5 % by weight based on the composition.

6. The method as claimed in Claim I, wherein said active ingredient is employed at a dosage of from I.0 to 0.0I mg/cm$^2$/day.

7. The method as claimed in Claim 6, wherein said active ingredient is employed at a dosage of from 0.5 to 0.05 mg/cm$^2$/day.

**Revendications**

1. Composition pour la repousse des cheveux contenant comme ingrédient actif un peptide représenté par la formule (I) :

$$His-Ser-Asp-Ala-Val-Phe-Thr-$$
$$Asp-Asn-Tyr-Thr-Arg-Leu-arg-\qquad (I)$$
$$Lys-Gln-Met-Ala-Val-Lys-Lys-$$
$$Tyr-Leu-Asn-Ser-Ile-Leu-Asn-NH_2$$

dans laquelle His, Ser, Asp, Ala, Val, Phe, Thr, Asn, Tyr, Arg, Leu, Lys, Gln, Met, et Ile représentent l'histidine, la sérine, l'acide aspartique, l'alanine, la valine, la phénylalanine, la thréonine, l'asparagine, la tyrosine, l'arginine, la leucine, la lysine, la glutamine, la méthionine, et l'isoleucine, respectivement, dans une quantité de 0,005 à 0,5 % en poids par rapport à la composition et un véhicule ou diluant acceptable pharmaceutiquement.

2. Composition pour la repousse des cheveux suivant la revendication 1, dans laquelle ce support est un fluide ou un semi-solide.

3. Composition pour la repousse des cheveux suivant la revendication 2, dans laquelle ce support est un semi-solide.

4. Composition pour la repousse des cheveux suivant la revendication 3, dans laquelle ce semi-solide est choisi parmi la paraffine liquide, le vaseline hydrophyle, la lanoline et le polyéthylène glycol.

5. Procédé pour augmenter la repousse des cheveux comprenant l'application locale sur la peau d'une composition pour la repousse des cheveux contenant un peptide représenté par la formule (I) :

His−Ser−Asp−Ala−Val−Phe−Thr−

Asp−Asn−Tyr−Thr−Arg−Leu−Arg−　　　(I)

Lys−Gln−Met−Ala−Val−Lys−Lys−

Tyr−Leu−Asn−Ser−Ile−Leu−Asn−NH$_2$

dans laquelle His, Ser, Asp, Ala, Val, Phe, Thr, Asn, Tyr, Arg, Leu, Lys, Gln, Met, et Ile représentent l'histidine, la sérine, l'acide aspartique, l'alanine, la valine, la phénylalanine, la thréonine, l'asparagine, la tyrosine, l'arginine, la leucine, la lysine, la glutamine, la méthionine, et l'isoleucine, respectivement, dans une quantité de 0,005 à 0,5 % en poids par rapport à la composition.

6. Procédé suivant la revendication 1, dans laquelle cet ingrédient actif est utilisé à une dose de 1,0 à 0,01 mg/cm²/jour.

7. Procédé suivant la revendication 6, dans lequel cet ingrédient actif est utilisé à une dose de 0,5 à 0,05 mg/cm²/jour.

**Ansprüche**

1. Haarwuchspräparat, das als aktives Mittel ein Peptid der Formel (I):

His−Ser−Asp−Ala−Val−Phe−Thr−

Asp−Asn−Tyr−Thr−Arg−Leu−arg−　　　(I)

Lys−Gln−Met−Ala−Val−Lys−Lys−

Tyr−Leu−Asn−Ser−Ile−Leu−Asn−NH$_2$

enthält, wobei His, Ser, Asp, Ala, Val, Phe, Thr, Asn, Tyr, Arg, Leu, Lys, Gln, Met und Ile für Histidin, Serin, Asparginsäure, Alanin, Valin, Phenylalanin, Threonin, Asparagin, Tyrosin, Arginin, Leucin, Lysin, Glutamin, Methionin bzw. Isoleucin stehen, wobei die Menge von 0,005 bis 0,5 Gew.%, bezogen auf das Präparat, beträgt, mit zusätzlichem Gehalt an einem pharmazeutisch verträglichen Träger oder Verdünnungsmittel.

2. Haarwuchspräparat gemäß Anspruch 1, wobei der Träger ein Fluid oder ein halbfester Träger ist.

3. Haarwuchspräparat nach Anspruch 2, gekennzeichnet durch einen halbfesten Träger.

4. Haarwuchspräparat nach Anspruch 3, wobei als halbfester Träger flüssiges Paraffin, hydrophiles Petrolat, Lanolin und/oder Polyethylenglycol verwendet wird.

5. Verfahren zur Förderung des Haarwuchses durch topikales Aufbringen eines Haarwuchspräparats mit Gehalt an einem Peptid der Formel (I)

```
His-Ser-Asp-Ala-Val-Phe-Thr-
Asp-Asn-Tyr-Thr-Arg-Leu-arg-        (I)
Lys-Gln-Met-Ala-Val-Lys-Lys-         .
Tyr-Leu-Asn-Ser-Ile-Leu-Asn-NH2
```

wobei His, Ser, Asp, Ala, Val, Phe, Thr, Asn, Tyr, Arg, Leu, Lys, Gln, Met und Ile für Histidin, Serin, Asparginsäure, Alanin, Valin, Phenylalanin, Threonin, Asparagin, Tyrosin, Arginin, Leucin, Lysin, Glutamin, Methionin bzw. Isoleucin stehen, wobei die Menge von 0,005 bis 0,5 Gew.%, bezogen auf das Präparat, beträgt.

6. Verfahren nach Anspruch 5, dadurch gekennzeichnet, daß die Wirkungskomponente in einer Dosierung von 1,0 bis 0,01/mg/cm²/Tag verwendet wird.

7. Verfahren nach Anspruch 6, dadurch gekennzeichnet, daß die Wirkungskomponente in einer Dosierung von 0,5 bis 0,05 mg/cm²/Tag verwendet wird.